# EUROPEAN PATENT APPLICATION

(11) **EP 4 803 137 A1**
(43) Date of publication of application: **09.09.2026**
(21) Application number: 26162652.7
(22) Date of filing: 05.03.2026
(51) Int. Cl.: A61N 5/10

(54) **METHOD AND APPARATUS TO FACILITATE OPTIMIZING A RADIATION TREATMENT PLAN**

(30) Priority: 07.03.2025 US 202519073663
(71) Applicant: Siemens Healthineers International AG, 6312 Steinhausen (CH)
(72) Inventor: DONNELLY, Kellee, 00100 Helsinki (FI); VALENZUELA, Daniel, 00100 Helsinki (FI)
(74) Representative: Mathisen & Macara LLP

(57) **Abstract**

To facilitate optimization of a treatment plan a control circuit 101 is configured to access 201 information regarding a particular patient 104 that identifies a volume 401 to be avoided during treatment along with accessing 202 information identifying a reference point as regards the particular patient, and then automatically identify 203 angles 404 from which the particular patient shall not be irradiated as a function of the volume to be avoided during treatment and the reference point.

## Description

### TECHNICAL FIELD

These teachings relate generally to treatment planning for a patient's planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Some patient cases require areas where no dose should enter. These areas are sometimes referred to as avoidance sectors within arc fields. In a typical prior art treatment planning system, such sectors are typically defined by the end user who leverages their own understanding of the arc field projections with respect to patient anatomy to identify particular angles (and corresponding sectors) where the radiation beam should be off. This human-oriented approach can be a very trial and error process, and is always necessarily based on a visual inspection by the end user.

### SUMMARY

In one aspect, the present invention provides a method to facilitate optimizing a radiation treatment plan for a particular patient wherein the radiation treatment plan includes planned irradiation of the particular patient from a plurality of different angles, as defined in claim 1. Optional features are specified in the dependent claims.

In another aspect, the present invention provides apparatus to facilitate optimizing a radiation treatment plan for a particular patient wherein the radiation treatment plan includes irradiating the particular patient from a plurality of different angles, as defined in claim 10. Optional features are specified in the dependent claims.

In a further aspect, the present invention provides a non-transitory computer-readable medium to facilitate optimizing a radiation treatment plan for a particular patient wherein the radiation treatment plan includes irradiating that particular patient from a plurality of different angles, comprising instructions stored thereon, that when executed on a processor, perform the steps as defined in claim 14. Optional features are specified in the dependent claim.

Some embodiments of the invention include accessing information regarding the particular patient that identifies a volume to be avoided during treatment.

The volume to be avoided may comprise or contain an artifact. The volume to be avoided may contain an entity that is prone to harm by application of radiation treatment. The entity may be an organ-at-risk, such as non-targeted patient tissues. The entity may be either proximal to the treatment volume or distal therefrom. The volume to be avoided during treatment may contain an object implanted in the patient or a foreign object. The object may be, for example, a pacemaker, implant (e.g. a hip implant that is implanted in the patient), an implanted drug dispenser, an implanted patient monitor, or a lens) or something similar that may e.g. be harmed in some way by energy/radiation. The object may be either proximal to the treatment volume or distal therefrom.

Some embodiments of the invention include accessing information regarding the particular patient that includes patient imaging views (such as a computed tomography field of view). The volume to be avoided may comprise or contain areas of the patient that are outside of the patient imaging views, such as one or both of the patient's shoulders.

### BRIEF DESCRIPTION OF DRAWINGS

Various needs are at least partially met through provision of the method and apparatus to facilitate optimizing a radiation treatment plan described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a flow diagram as configured in accordance with various embodiments of these teachings;
FIG. 4 comprises a schematic view as configured in accordance with various embodiments of these teachings;
FIG. 5 comprises a schematic view as configured in accordance with various embodiments of these teachings; and
FIG. 6 comprises a schematic view as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments can serve to facilitate optimizing a radiation treatment plan for a particular patient wherein the radiation treatment plan includes irradiating the particular patient from a plurality of different angles. In such a case, irradiating the particular patient can comprise using a radiation source that moves along an arcuate pathway around the particular patient.

By one approach, the control circuit accesses information regarding the particular patient that identifies the volume to be avoided during treatment. Such a volume can comprise a part of the patient themselves or can comprise, for example, an artifact. The control circuit can also access information identifying a reference point, such as an isocenter, as regards the particular patient. The control circuit can then automatically identify angles from which the particular patient shall not be irradiated as a function of the volume to be avoided during treatment and that reference point. By one approach, the identification of angles can comprise identifying arcuate portions of the aforementioned arcuate pathway.

By one approach, automatically identifying angles from which the particular patient shall not be irradiated can comprise projecting lines from the reference point to an arcuate pathway that includes the plurality of different angles. The latter may comprise, for example, projecting the lines to form a section on the arcuate pathway that conformally includes the volume to be avoided. If desired, these teachings will accommodate adding a predetermined margin to at least one side of the section on the arcuate pathway to thereby increase the identified angles from which the particular patient shall not be irradiated.

By one approach, the control circuit can then optimize a radiation treatment plan for the particular patient while avoiding the angles from which the particular patient shall not be irradiated to provide an optimized radiation treatment plan. The optimized radiation treatment plan can then optionally be used to administer therapeutic radiation to the particular patient.

So configured, these teachings will allow a user to indicate the areas of avoidance they wish to see with respect to the patient anatomy, and these teachings will provide for automatically projecting lines from a suitable reference point to define a corresponding respective portion of the arc where the radiation source shall be off when traversing that area.

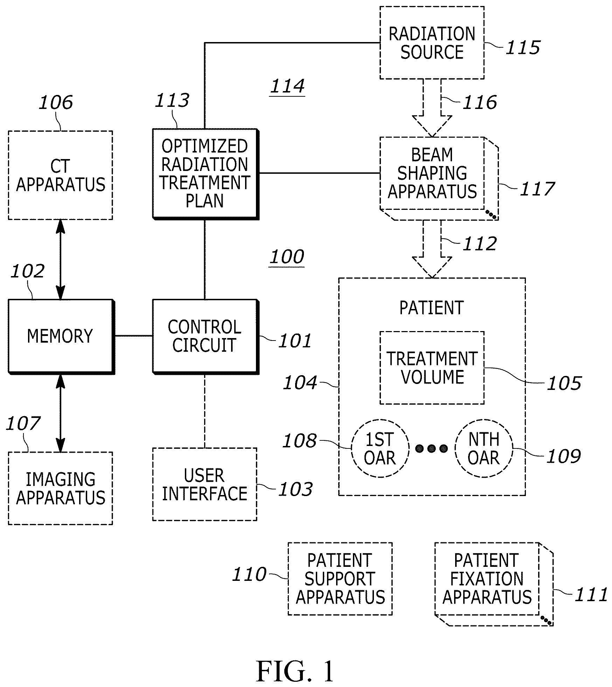

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 is beam-on and beam-off, when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

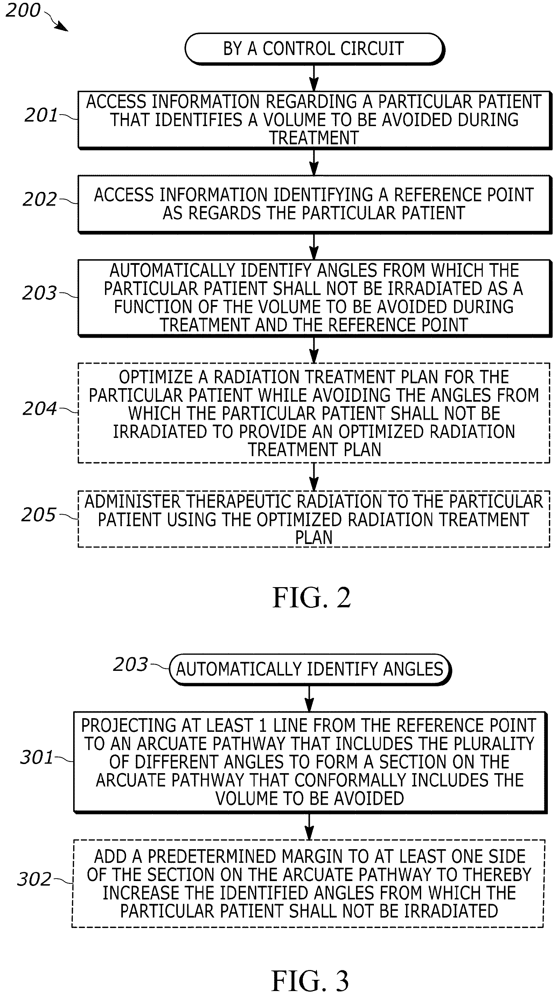

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate optionally treating a particular patient with therapeutic radiation using a particular radiation treatment platform per that optimized radiation treatment plan, and more particularly facilitating the optimization of a radiation treatment plan for a particular patient wherein the radiation treatment plan includes irradiating the particular patient from a plurality of different angles (as may occur, for example, when irradiating the particular patient using a radiation source 115 that moves along an arcuate pathway around the particular patient).

At block 201, the control circuit 101 accesses information regarding the particular patient 104 that identifies a volume to be avoided during treatment. This volume to be avoided during treatment may be an organ-at-risk 108, such as non-targeted patient tissues that are proximal to the intended treatment volume 105. In this context, this volume to be avoided during treatment may not merely be a typical organ-at-risk 108, such as non-targeted patient tissues that are proximal to the intended treatment volume 105. A non-exclusive listing of examples of volumes to be avoided during treatment in this context include, but are not limited to, artifacts (such as, for example, a pacemaker or metal hip implants that are implanted in the patient, either proximal to the treatment volume 105 or distal therefrom, an implanted drug dispenser, an implanted patient monitor, or a lens) that may be harmed in some way by energy/radiation, areas of the patient 104 that were outside of patient imaging views (such as a computed tomography field of view), such as one or both of the patient's shoulders, that are being relied upon for radiation treatment planning purposes, and so forth.

At block 202, the control circuit 101 also accesses information identifying a reference point as regards the particular patient. Although this reference point can be arbitrarily established if desired, in many application settings it can be useful for the reference point to comprise an isocenter that corresponds to the application setting. Those skilled in the art of radiotherapy know that an isocenter is the point in space through which the center of a beam of radiation passes, especially when the beam of radiation moves with respect to the patient. In many cases, the isocenter is the point in space relative to a radiation treatment platform about which a source of radiation rotates via a gantry and hence is essentially defined by the geometry of the radiation treatment platform itself.

By one approach, if desired, one or both of the information regarding the particular patient that identifies a volume to be avoided during treatment and the information that identifies a reference point as regards the particular patient can comprise user-entered information. Such information could be entered, for example, via the aforementioned user interface 103. To identify a volume to be avoided, for example, a user might selected a segmented volume on a patient image, or might draw an area to be so avoided on patient imagery.

At block 203, the control circuit 101 then automatically identifies angles from which the particular patient 104 shall not be irradiated as a function of both the reference point and the aforementioned volume to be avoided during treatment. In a not untypical application setting, this activity can comprise automatically identifying specific arcuate portions of the arcuate pathway upon which the radiation source 115 circles the particular patient 104 during treatment.

Referring now as well to FIG. 3, one illustrative approach to automatically identifying the aforementioned angles includes, at box 301, projecting one or more lines from the aforementioned reference point to an arcuate pathway that includes the aforementioned plurality of different angles. This activity can include projecting such lines to form one or more sections on the arcuate pathway that conformally includes the aforementioned volume to be avoided.

As illustrated at optional block 302, these teachings will accommodate adding a predetermined margin to at least one side of the aforementioned section on the arcuate pathway to thereby increase the identified angle or angles from which the particular patient shall not be irradiated. Such a margin can be a set predetermined value such as 0.25°, 0.5°, 1.0°, 5.0°, or any value within a given range such as, a range of 0.1° to 5.0° as desired. It will be appreciated that the margin can be specified as an angle (as in the examples above) or as a linear distance (such as millimeters) from the structure to avoid. The latter approach may better correspond to certain location uncertainties due, for example, to patient movement. If desired, these teachings will also accommodate using different margins at different portions of the overall arcuate pathway. For example, a first margin value may apply during a first specified portion of the overall arcuate pathway while a second margin value is applied during a second, subsequent specified portion of the overall arcuate pathway.

Referring again to FIG. 2, at optional block 204 the control circuit 101 can then optimize a radiation treatment plan for the particular patient 104 while avoiding the angles from which the particular patient 104 shall not be irradiated to provide an optimized radiation treatment plan 113. At optional block 205, the control circuit 101 can then administer therapeutic radiation to the particular patient 104 using that optimized radiation treatment plan 113.

So configured, these teachings provide for allowing a user to indicate one or more areas of avoidance they wish to see with respect to the patient anatomy, and the control circuit 101 can project this indicated area to define one or more corresponding respective portions of the gantry arc where the beam should be off during administration of the treatment plan.

Further details that comport with these teachings will now be presented. It will be understood that the specific details of these examples are intended to serve an illustrative purpose and are not intended to suggest any particular limitations with respect to these teachings.

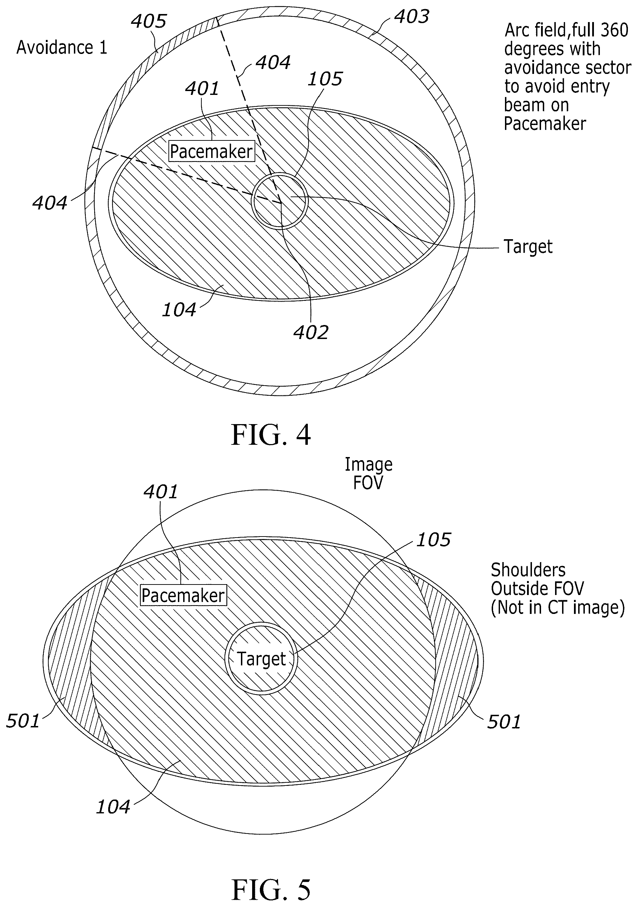

FIG. 4 presents an illustrative example where the patient 104 has an embedded object such as a pacemaker 401 (or other foreign body). From the perspective of the aforementioned reference point 402 (in this example, the isocenter) and the full arc field 403, these teachings provide for automatically identifying the conforming bounding angles 404 that conformally define therebetween an avoidance sector 405 within which the radiation beam should not impinge even when application of a radiation beam towards the treatment target 105 might otherwise be desirable.

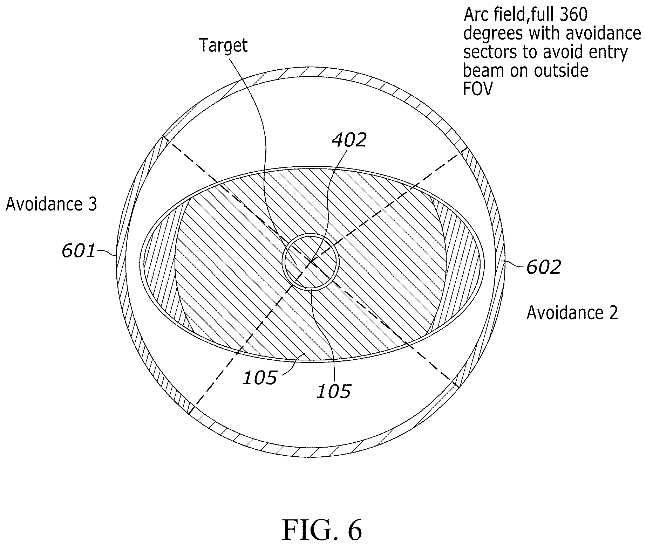

FIG. 5 presents an example where the patient's shoulders 501 (or other body part) were outside the field of view of e.g. the computed tomography imaging being used for imaging purposes. In this case, and referring to FIG. 6, these teachings provide for generating a first avoidance sector 601 and a second avoidance sector 602 to accommodate those unknown patient volumes (here, the patient's shoulders). For example, an additional margin can be added on either (or both) side to somewhat expand one or both avoidance sectors 601 and 602.

If the patient 104 has an embedded pacemaker 401 (or other foreign body) and the patient's shoulders 501 (or other body part) were outside the field of view of the computed tomography imaging being used for imaging purposes, then these teachings provide for automatically identifying the conforming bounding angles 404 that conformally define therebetween an avoidance sector 405 ("Avoidance 1" relating to the pacemaker), and avoidance sector 601 ("Avoidance 3") and avoidance sector 602 ("Avoidance 2") to accommodate those unknown patient volumes.

By one approach, these teachings can comprise a computer program that itself comprises instructions that, when the computer program is executed by a computer, causes the computer to carry out any or all of the aforementioned steps, functions, and/or activities.

Further aspects of these teachings are provided by the subject matter of the following clauses (where it will be understood that any of these clauses can be combined with any one of more of the other clauses as appropriate).

Clause 1. A method to facilitate optimizing a radiation treatment plan for a particular patient wherein the radiation treatment plan includes irradiating the particular patient from a plurality of different angles, the method comprising: by a control circuit: accessing information regarding the particular patient that identifies a volume to be avoided during treatment; accessing information identifying a reference point as regards the particular patient; automatically identifying angles from which the particular patient shall not be irradiated as a function of the volume to be avoided during treatment and the reference point.

Clause 2. The method of clause 1 wherein irradiating the particular patient from a plurality of different angles comprises irradiating the particular patient using a radiation source that moves along an arcuate pathway around the particular patient.

Clause 3. The method of clause 2 wherein automatically identifying angles from which the particular patient shall not be irradiated comprises automatically identifying arcuate portions of the arcuate pathway.

Clause 4. The method of clause 1 wherein accessing information regarding the particular patient that identifies a volume to be avoided during treatment comprises accessing user-entered information.

Clause 5. The method of clause 1 wherein the volume to be avoided comprises an artifact.

Clause 6. The method of clause 1 wherein the reference point comprises an isocenter.

Clause 7. The method of clause 1 wherein automatically identifying angles from which the particular patient shall not be irradiated comprises: projecting lines from the reference point to an arcuate pathway that includes the plurality of different angles.

Clause 8. The method of clause 7 wherein projecting the lines from the reference point to the arcuate pathway comprises projecting the lines to form a section on the arcuate pathway that conformally includes the volume to be avoided.

Clause 9. The method of clause 8 further comprising: adding a predetermined margin to at least one side of the section on the arcuate pathway to thereby increase the identified angles from which the particular patient shall not be irradiated.

Clause 10. The method of clause 1 further comprising: optimizing a radiation treatment plan for the particular patient while avoiding the angles from which the particular patient shall not be irradiated to provide an optimized radiation treatment plan; administering therapeutic radiation to the particular patient using the optimized radiation treatment plan.

Clause 11. An apparatus to facilitate optimizing a radiation treatment plan for a particular patient wherein the radiation treatment plan includes irradiating the particular patient from a plurality of different angles, the apparatus comprising: a control circuit configured to: access information regarding the particular patient that identifies a volume to be avoided during treatment; access information identifying a reference point as regards the particular patient; automatically identify angles from which the particular patient shall not be irradiated as a function of the volume to be avoided during treatment and the reference point.

Clause 12. The apparatus of clause 11 wherein irradiating the particular patient from a plurality of different angles comprises irradiating the particular patient using a radiation source that moves along an arcuate pathway around the particular patient.

Clause 13. The apparatus of clause 12 wherein the control circuit is configured to automatically identify angles from which the particular patient shall not be irradiated by automatically identifying arcuate portions of the arcuate pathway.

Clause 14. The apparatus of clause 11 wherein the control circuit is configured to access information regarding the particular patient that identifies a volume to be avoided during treatment by accessing user-entered information.

Clause 15. The apparatus of clause 11 wherein the volume to be avoided comprises an artifact.

Clause 16. The apparatus of clause 11 wherein the reference point comprises an isocenter.

Clause 17. The apparatus of clause 11 wherein the control circuit is configured to automatically identify angles from which the particular patient shall not be irradiated by: projecting lines from the reference point to an arcuate pathway that includes the plurality of different angles.

Clause 18. The apparatus of clause 17 wherein the control circuit is configured to project the lines from the reference point to the arcuate pathway by projecting the lines to form a section on the arcuate pathway that conformally includes the volume to be avoided.

Clause 19. The apparatus of clause 18 wherein the control circuit is further configured to: add a predetermined margin to at least one side of the section on the arcuate pathway to thereby increase the identified angles from which the particular patient shall not be irradiated.

Clause 20. The apparatus of clause 11 wherein the control circuit is further configured to: optimize a radiation treatment plan for the particular patient while avoiding the angles from which the particular patient shall not be irradiated to provide an optimized radiation treatment plan; administer therapeutic radiation to the particular patient using the optimized radiation treatment plan.

Clause 21. A non-transitory computer-readable medium to facilitate optimizing a radiation treatment plan for a particular patient wherein the radiation treatment plan includes irradiating that particular patient from a plurality of different angles, comprising instructions stored thereon, that when executed on a processor, perform the steps of: accessing information regarding the particular patient that identifies a volume to be avoided during treatment; accessing information identifying a reference point as regards the particular patient; automatically identifying angles from which the particular patient shall not be irradiated as a function of the volume to be avoided during treatment and the reference point.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method to facilitate optimizing a radiation treatment plan for a particular patient wherein the radiation treatment plan includes planned irradiation of the particular patient from a plurality of different angles, the method comprising:
by a control circuit:
accessing information regarding the particular patient that identifies a volume to be avoided during treatment;
accessing information identifying a reference point as regards the particular patient;
automatically identifying angles from which the particular patient shall not be irradiated as a function of the volume to be avoided during treatment and the reference point.

2. The method of claim 1 wherein the planned irradiation of the particular patient from a plurality of different angles comprises planned irradiation of the particular patient using a radiation source that moves along an arcuate pathway around the particular patient.

3. The method of claim 2 wherein automatically identifying angles from which the particular patient shall not be irradiated comprises automatically identifying arcuate portions of the arcuate pathway.

4. The method of claim 1, 2 or 3 wherein accessing information regarding the particular patient that identifies a volume to be avoided during treatment comprises accessing user-entered information.

5. The method of any one of claims 1 to 4 wherein the volume to be avoided comprises an artifact.

6. The method of any one of claims 1 to 5 wherein the reference point comprises an isocenter.

7. The method of any one of claims 1 to 6 wherein automatically identifying angles from which the particular patient shall not be irradiated comprises:
projecting lines from the reference point to an arcuate pathway that includes the plurality of different angles.

8. The method of claim 7 wherein projecting the lines from the reference point to the arcuate pathway comprises projecting the lines to form a section on the arcuate pathway that conformally includes the volume to be avoided.

9. The method of claim 8 further comprising:
adding a predetermined margin to at least one side of the section on the arcuate pathway to thereby increase the identified angles from which the particular patient shall not be irradiated.

10. An apparatus to facilitate optimizing a radiation treatment plan for a particular patient wherein the radiation treatment plan includes irradiating the particular patient from a plurality of different angles, the apparatus comprising:
a control circuit configured to:
access information regarding the particular patient that identifies a volume to be avoided during treatment;
access information identifying a reference point as regards the particular patient;
automatically identify angles from which the particular patient shall not be irradiated as a function of the volume to be avoided during treatment and the reference point.

11. The apparatus of claim 10 wherein irradiating the particular patient from a plurality of different angles comprises irradiating the particular patient using a radiation source that moves along an arcuate pathway around the particular patient, and, optionally, wherein the control circuit is configured to automatically identify angles from which the particular patient shall not be irradiated by automatically identifying arcuate portions of the arcuate pathway.

12. The apparatus of claim 10 wherein:
the volume to be avoided comprises an artifact;
the reference point comprises an isocenter; and/or
the control circuit is configured to perform the method of any one of claims 4, 7, 8 or 9.

13. The apparatus of claim 10, 11 or 12 wherein the control circuit is further configured to:
optimize a radiation treatment plan for the particular patient while avoiding the angles from which the particular patient shall not be irradiated to provide an optimized radiation treatment plan;
administer therapeutic radiation to the particular patient using the optimized radiation treatment plan.

14. A non-transitory computer-readable medium to facilitate optimizing a radiation treatment plan for a particular patient wherein the radiation treatment plan includes irradiating that particular patient from a plurality of different angles, comprising instructions stored thereon, that when executed on a processor, perform the steps of:
accessing information regarding the particular patient that identifies a volume to be avoided during treatment;
accessing information identifying a reference point as regards the particular patient;
automatically identifying angles from which the particular patient shall not be irradiated as a function of the volume to be avoided during treatment and the reference point.

15. The non-transitory computer-readable medium of claim 14, wherein the instructions, when executed on a processor, perform the method of any one of claims 1 to 9.
